# EUROPEAN PATENT APPLICATION

(11) **EP 4 785 982 A1**
(43) Date of publication of application: **05.08.2026**
(21) Application number: 24871435.4
(22) Date of filing: 03.07.2024
(51) Int. Cl.: A61M 25/09, A61M 25/092

(54) **OPERATION MEMBER FOR GUIDE WIRE**

(30) Priority: 29.09.2023 JP 2023169659
(71) Applicant: NHK Spring Co., Ltd., Yokohama-shi, Kanagawa 236-0004 (JP)
(72) Inventor: UETSUHARA, Saiji, Yokohama-shi, Kanagawa 236-0004 (JP); NAKAZONO, Miho, Yokohama-shi, Kanagawa 236-0004 (JP)
(74) Representative: V.O.
(86) International application number: PCT/JP2024/024013
(87) International publication number: WO 2025/069631

(57) **Abstract**

A first tubular member that extends in a front-rear direction, a second tubular member that extends in the front-rear direction, and an outer tube that is rotatably fitted around the first tubular member and the second tubular member about an axis and extends in the front-rear direction and that extends in the front-rear direction are provided, the first tubular member and the second tubular member are provided to be movable relative to each other in the front-rear direction and to be restricted from rotating relative to each other around the axis, the outer tube is provided on any one of the first tubular member and the second tubular member in a state where movement in the front-rear direction is restricted, and a first guide portion is formed on an inner peripheral surface of the outer tube and on an outer peripheral surface of the other one of the first tubular member and the second tubular member, the first guide portion being configured, in accordance with relative rotation of the outer tube and the other tubular member about the axis, to move the other tubular member in the front-rear direction relative to the one tubular member such that a distance in the front-rear direction between the fixation body and a front end portion of the support tube body in a guide wire changes.

## Description

### TECHNICAL FIELD

The present invention relates to an operation member for a guide wire.

Priority is claimed on Japanese Patent Application No. 2023-169659, filed September 29, 2023, the content of which is incorporated herein by reference.

### BACKGROUND ART

In the related art, for example, as shown in Patent Document 1, an operation member for deforming a front end portion of a guide wire is known. In general, the guide wire is inserted into a blood vessel and is used to guide a catheter for angiography to a chronic total occlusion lesion or the like in a state of being inserted through the chronic total occlusion lesion or the like in the blood vessel.

The operation member for a guide wire shown in Patent Document 1 includes a first tubular member and a second tubular member that extend in a front-rear direction, and an outer tube that is rotatably fitted around the first tubular member and the second tubular member and that extends in the front-rear direction. The first tubular member and the second tubular member are provided to be movable relative to each other in the front-rear direction and to be restricted from rotating relative to each other. A guide portion that causes the second tubular member to move in the front-rear direction with respect to the first tubular member in accordance with relative rotation of the outer tube and the second tubular member is formed on an inner peripheral surface of the outer tube and an outer peripheral surface of the second tubular member.

### Citation List

### Patent Document

Patent Document 1: Japanese Patent No. 7188847

### SUMMARY OF INVENTION

### Technical Problem

In the operation member for a guide wire in the related art, a relative position between the outer tube and the first tubular member in the front-rear direction is unstable, and there is a possibility that, for example, the outer tube moves in a front-rear direction with respect to the first tubular member or the second tubular member does not move in a front-rear direction with respect to the first tubular member when the outer tube is rotated with respect to the first tubular member and the second tubular member. In this case, the front end portion of the guide wire cannot be accurately deformed into a desired shape.

The present invention provides an operation member for a guide wire that can accurately deform a front end portion of the guide wire into a desired shape.

### Solution to Problem

According to an aspect of the present invention, there is provided an operation member for a guide wire, the operation member being mounted on a guide wire including a flexible tube body extending in a front-rear direction, an operation wire extending in the front-rear direction and provided inside the flexible tube body, a fixation body to which a front end edge of the operation wire is fixed, and a support tube body that is provided on a rear side of the fixation body, that sandwiches the flexible tube body in the front-rear direction between the fixation body and the support tube body, and that has the operation wire inserted therethrough, the operation member including a first tubular member that is fixed to a rear end portion of the support tube body and that extends in the front-rear direction; a second tubular member that is connected to a portion of the operation wire that protrudes rearward from the support tube body, the second tubular member extending in the front-rear direction; and an outer tube that is rotatably fitted around the first tubular member and the second tubular member about an axis and that extends in the front-rear direction, the outer tube extending in the front-rear direction, in which the first tubular member and the second tubular member are provided to be movable relative to each other in the front-rear direction and to be restricted from rotating relative to each other around the axis, the outer tube is provided on any one of the first tubular member and the second tubular member in a state where movement in the front-rear direction is restricted, and a first guide portion is formed on an inner peripheral surface of the outer tube and on an outer peripheral surface of the other one of the first tubular member and the second tubular member, the first guide portion being configured, in accordance with relative rotation of the outer tube and the other tubular member about the axis, to move the other tubular member in the front-rear direction relative to the one tubular member such that a distance in the front-rear direction between the fixation body and a front end portion of the support tube body changes.

In a case where the outer tube that is provided on any one of the first tubular member and the second tubular member in a state where movement in the front-rear direction is restricted is rotated around the axis, the other tubular member moves in the front-rear direction with respect to the one tubular member by the first guide portion formed on the inner peripheral surface of the outer tube and the outer peripheral surface of the other one of the first tubular member and the second tubular member. In this case, the distance between the fixation body and the front end portion of the support tube body in the front-rear direction is shortened, and the flexible tube body is compressed and deformed.

As a result, for example, when an eccentric load is generated in the flexible tube body in a case where the flexible tube body is a coil spring, or in a case where the central axes of each of the flexible tube body and the operation wire do not coincide with one another, the flexible tube body is compressed and deformed while being bent in a specific direction, and the outer tube is operated outside the body to select a blood vessel at a blood vessel bifurcation while bending the front end portion of the guide wire in the body, so that the guide wire can enter a target blood vessel. As a result, it is possible to reduce reliance on a physician's tactile sense during manipulation when advancing the guide wire along the blood vessel to reach a chronic total occlusion lesion or the like. In addition, it is possible to eliminate a need to finely adjust a curvature of the front end portion of the guide wire outside the body by repeatedly inserting and removing the guide wire into and from the blood vessel.

Further, when the outer tube is rotated around the axis, the distance between the fixation body and the front end portion of the support tube body in the front-rear direction is shortened, and the flexible tube body is compressed and deformed in the front-rear direction, the bending stiffness of the flexible tube body is increased. Therefore, the bending stiffness of the flexible tube body can be changed by operating the outer tube outside the body in a state where the guide wire is inserted into the blood vessel.

As a result, when penetrating the chronic total occlusion lesion or the like with the guide wire, the bending stiffness of the flexible tube body can be kept low until the guide wire enters the blood vessel and reaches the chronic total occlusion lesion or the like in the blood vessel, and the outer tube can be rotated around the axis when the chronic total occlusion lesion or the like is punctured with the guide wire so as to penetrate the chronic total occlusion lesion or the like, whereby the bending stiffness of the flexible tube body can be increased, and the outer tube can be rotated in reverse after the chronic total occlusion lesion or the like is penetrated to thereby return the bending stiffness of the flexible tube body to the original low state. Therefore, it is possible to eliminate the need to repeatedly insert, remove, and exchange guide wires having different bending stiffnesses into and from a blood vessel or the like in order to penetrate the chronic total occlusion lesion or the like.

As described above, it is possible to shorten an operation time.

In the above process, the movement of the outer tube in the front-rear direction with respect to the one tubular member is restricted, and the first guide portion is formed on the inner peripheral surface of the outer tube and the outer peripheral surface of the other tubular member. Therefore, when the outer tube is rotated around the axis, the movement of the outer tube in the front-rear direction with respect to the one tubular member is restricted, and the other tubular member can be reliably moved in the front-rear direction with respect to the one tubular member, so that the front end portion of the guide wire can be accurately deformed into a desired shape.

The design of bending the flexible tube body in a specific direction when the outer tube is rotated around the axis can be performed, for example, by adjusting an eccentric amount, an eccentric direction, a tilt angle, and the like of the central axis of each of the flexible tube body and the operation wire, or by adjusting an eccentric amount, an eccentric direction, and the like of a load generated in the flexible tube body when a compression load is applied in a case where the flexible tube body is a coil spring. The latter adjustment can be performed, for example, by adjusting a spring shape such as the number of turns of the coil spring (relative circumferential positions of both end portions of the wire material), a pitch angle of the coil spring, a pitch of the coil spring, a wire diameter (diameter of the wire material), a gap between the wires, a coil outer diameter, and a support method of both end portions of the coil spring (for example, inclinations of both end portions of the wire material).

The first guide portion may include a first engagement protrusion and a first guide groove that extends around the axis and in which the first engagement protrusion is movably accommodated around the axis, and a second guide portion that allows relative movement in the front-rear direction may be formed in the first tubular member and the second tubular member in a state where the first tubular member and the second tubular member are restricted from rotating relative to each other around the axis, the second guide portion may include a second engagement protrusion and a second guide groove that extends in the front-rear direction and in which the second engagement protrusion is movably accommodated in the front-rear direction, and the first guide portion and the second guide portion may be separated from each other in the front-rear direction.

The first guide portion and the second guide portion are separated from each other in the front-rear direction, the first engagement protrusion and the second engagement protrusion are individually accommodated in the first guide groove and the second guide groove, and are not integrally accommodated in the first guide groove and the second guide groove. Therefore, it is possible to suppress the amount of protrusion of each of the first engagement protrusion and the second engagement protrusion. In addition, compared to a configuration in which the first engagement protrusion or the second engagement protrusion is integrally accommodated in the first guide groove and the second guide groove, the first engagement protrusion and the second engagement protrusion can be easily and individually accommodated in the first guide groove and the second guide groove, without excessively radially elastically deforming the first tubular member or the second tubular member during assembly, and the stiffness of each of the first tubular member and the second tubular member can be easily ensured. Advantageous Effects of Invention

According to the aspect of the present invention, the front end portion of the guide wire can be accurately deformed into a desired shape.

### BRIEF DESCRIPTION OF DRAWINGS

[FIG. 1] A longitudinal cross-sectional view showing a state where an operation member for a guide wire according to an embodiment is mounted on a guide wire.
[FIG. 2] A view showing a state where an outer tube of the operation member for a guide wire of FIG. 1 is rotated with respect to a second tubular member and the second tubular member is moved rearward with respect to a first tubular member.
[FIG. 3] A longitudinal cross-sectional view showing a state where an operation member for a guide wire according to a modification example is mounted on a guide wire.
[FIG. 4] A longitudinal cross-sectional view showing a state where the operation member for a guide wire according to the modification example is mounted on the guide wire.

### DESCRIPTION OF EMBODIMENTS

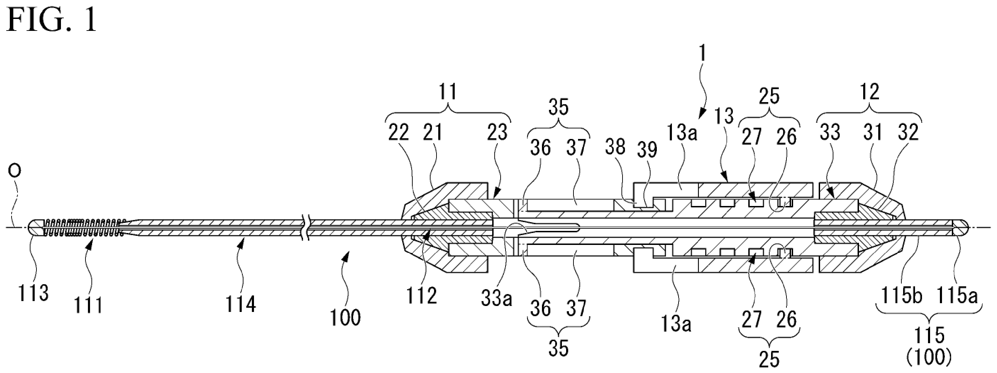

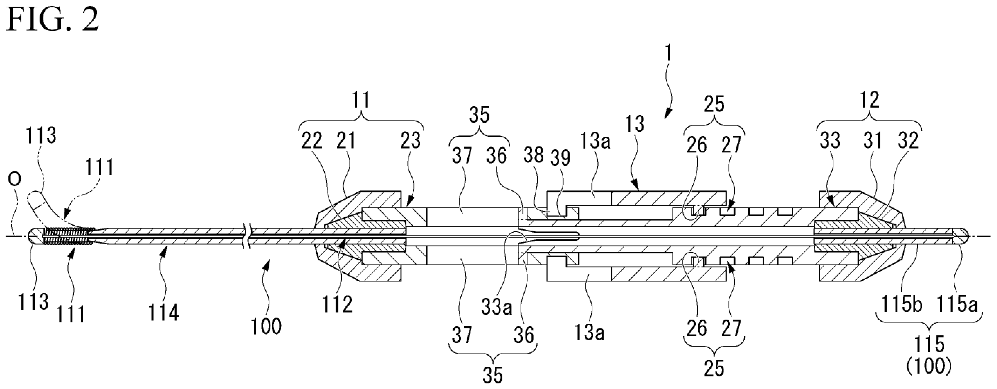

Hereinafter, a first embodiment of the operation member for a guide wire will be described with reference to FIGS. 1 and 2.

First, a guide wire 100 on which the operation member for a guide wire is mounted will be described. The form of the guide wire is not limited to the present embodiment and may be appropriately changed.

The guide wire 100 includes a flexible tube body 111, an operation wire 112, a front fixation body 113 (fixation body), a support tube body 114, and a rear fixation body 115. The flexible tube body 111, the operation wire 112, the front fixation body 113, the support tube body 114, and the rear fixation body 115 are formed of, for example, a metal material or the like. The material may be appropriately changed.

The flexible tube body 111, the operation wire 112, and the support tube body 114 are disposed coaxially with a common axis.

Hereinafter, the common axis will be referred to as a central axis O (axis), a side of the front fixation body 113 along the central axis O will be referred to as a front side, a side of the support tube body 114 along the central axis O will be referred to as a rear side, and a direction along the central axis O will be referred to as a front-rear direction. A direction intersecting the central axis O as viewed in the front-rear direction will be referred to as a radial direction, and a direction that revolves around the central axis O as viewed in the front-rear direction will be referred to as a circumferential direction.

The central axes of the flexible tube body 111, the operation wire 112, and the support tube body 114 need not coincide with one another, for example, may be inclined, may intersect, or may be separated from one another.

An outer diameter of the flexible tube body 111 is a size that can be inserted into a blood vessel (for example, 0.2 mm or more and 1.0 mm or less). A diameter of the wire material forming the flexible tube body 111 is the same over the entire length. An outer shape and a size of the flexible tube body 111 as viewed in the front-rear direction are the same over the entire length in the front-rear direction.

The diameter of the wire material forming the flexible tube body 111 need not be the same over the entire length, and the outer shape and the size of the flexible tube body 111 as viewed in the front-rear direction need not be the same over the entire length in the front-rear direction. For example, the outer diameter of the flexible tube body 111 may decrease toward the front.

The flexible tube body 111 extends in the front-rear direction and is formed to be bendable. The flexible tube body 111 is a coil spring extending in the front-rear direction. In the flexible tube body 111, bending stiffnesses of a front portion and a rear portion are smaller than a bending stiffness of an intermediate portion. When a compression load in the front-rear direction is applied to the flexible tube body 111, the front portion and the rear portion are reduced more than the intermediate portion in the front-rear direction.

The flexible tube body 111 may be a tubular body consisting of a circumferential wall that continuously extends over the entire length in each of the front-rear direction and the circumferential direction. In addition, the bending stiffness of the flexible tube body 111 may be the same over the entire length in the front-rear direction.

The operation wire 112 extends in the front-rear direction and is provided inside the flexible tube body 111. The operation wire 112 is formed to be elastically deformable. A plurality of operation wires 112 may be provided at positions spaced apart from the central axis O in the radial direction at intervals in the circumferential direction.

A front end edge of the operation wire 112 is fixed to the front fixation body 113. The front fixation body 113 is formed in a hemispherical shape having a convex spherical surface facing forward and a flat rear surface facing rearward. The front fixation body 113 is disposed coaxially with the central axis O. The front end edge of the operation wire 112 is fixed to the rear surface of the front fixation body 113 by, for example, brazing or crimping. A front end portion of the flexible tube body 111 abuts against the rear surface of the front fixation body 113. The front end portion of the flexible tube body 111 may be fixed to the rear surface of the front fixation body 113 by, for example, brazingor soldering.

Outer shapes and sizes of each of the front fixation body 113 and the flexible tube body 111 as viewed in the front-rear direction are the same as each other. The outer shapes and the sizes of each of the front fixation body 113 and the flexible tube body 111 as viewed in the front-rear direction may be different from each other. In this case, it is preferable that, as viewed in the front-rear direction, the flexible tube body 111 does not protrude radially outward from the front fixation body 113.

The support tube body 114 is provided behind the front fixation body 113. The flexible tube body 111 is sandwiched in the front-rear direction between the support tube body 114 and the front fixation body 113. The support tube body 114 extends in the front-rear direction. The operation wire 112 is inserted into the support tube body 114. The operation wire 112 penetrates the support tube body 114 in the front-rear direction.

A front portion of the support tube body 114 is reduced in diameter toward the front. A front end portion of the front portion of the support tube body 114 is inserted into a rear end portion of the flexible tube body 111. An outer peripheral surface of the front portion of the support tube body 114 is in contact with the rear end portion of the flexible tube body 111 in the front-rear direction and supports the rear end portion of the flexible tube body 111.

Outer shapes and sizes of each of a rear portion of the support tube body 114, which is located behind the front portion, and the flexible tube body 111 as viewed in the front-rear direction are the same as each other. The outer shapes and the sizes of each of the rear portion of the support tube body 114 and the flexible tube body 111 as viewed in the front-rear direction may be different from each other. In this case, it is preferable that, as viewed in the front-rear direction, the flexible tube body 111 does not protrude radially outward from an outer peripheral surface of the rear portion of the support tube body 114.

The rear fixation body 115 is provided behind the support tube body 114. A rear end edge of the operation wire 112 is fixed to the rear fixation body 115. The rear fixation body 115 includes an end wall portion 115a and a circumferential wall portion 115b that extends from an outer peripheral edge portion of the end wall portion 115a toward the front. The end wall portion 115a has the same shape as the front fixation body 113 and is provided in an orientation in the front-rear direction opposite to that of the front fixation body 113. The rear fixation body 115 is disposed coaxially with the central axis O.

The rear end edge of the operation wire 112 is fixed to a front surface of the end wall portion 115a by, for example, brazing or crimping. The circumferential wall portion 115b surrounds a rear end portion of the operation wire 112 from the radial outer side.

Next, an operation member 1 for a guide wire will be described.

The operation member 1 for a guide wire includes a first tubular member 11, a second tubular member 12, and an outer tube 13. The first tubular member 11, the second tubular member 12, and the outer tube 13 are disposed coaxially with the central axis O.

The first tubular member 11 is fixed to a rear end portion of the support tube body 114 and extends in the front-rear direction. The rear end portion of the support tube body 114 is fitted into a front end opening of the first tubular member 11.

In the example shown in the drawing, the first tubular member 11 includes an outer sleeve 21, an inner sleeve 22, and a first body tube 23. The inner sleeve 22 and the first body tube 23 may be integrally formed.

A front portion of the inner sleeve 22 is fitted into a front portion of the outer sleeve 21. An inner diameter of the front portion of the outer sleeve 21 and an outer diameter of the front portion of the inner sleeve 22 decrease toward the front. A plurality of slits (not shown) that penetrate the inner sleeve 22 in the radial direction are formed over the entire length in the front-rear direction, excluding the rear end portion, at intervals in the circumferential direction. A front end portion of the first body tube 23 is inserted between an inner peripheral surface of a rear portion of the outer sleeve 21 and an outer peripheral surface of a rear portion of the inner sleeve 22. The first body tube 23 protrudes rearward from the outer sleeve 21 and the inner sleeve 22. A male screw portion (not shown) is formed on an outer peripheral surface of the front end portion of the first body tube 23, and a female screw portion (not shown) that is screwed to the male screw portion of the first body tube 23 is formed on an inner peripheral surface of a rear portion of the outer sleeve 21.

The rear end portion of the support tube body 114 is fitted and fixed into the inner sleeve 22 through a front end opening of the outer sleeve 21. By tightening the outer sleeve 21 onto the first body tube 23, a portion of the inner sleeve 22, excluding the rear end portion, is reduced in diameter, and the inner peripheral surface of the inner sleeve 22 clamps the outer peripheral surface of the rear end portion of the support tube body 114.

The second tubular member 12 is connected to a portion of the operation wire 112 that protrudes rearward from the support tube body 114 and extends in the front-rear direction.

In the example shown in the drawing, the circumferential wall portion 115b of the rear fixation body 115 is fitted and fixed into a rear end opening of the second tubular member 12. As a result, the second tubular member 12 is connected to the operation wire 112 via the rear fixation body 115. The operation wire 112 may be directly connected to the second tubular member 12.

The second tubular member 12 includes an outer sleeve 31, an inner sleeve 32, and a second body tube 33. The inner sleeve 32 and the second body tube 33 may be integrally formed.

A rear portion of the inner sleeve 32 is fitted into a rear portion of the outer sleeve 31. An inner diameter of the rear portion of the outer sleeve 31 and an outer diameter of the rear portion of the inner sleeve 32 decrease toward the rear. A plurality of slits (not shown) that penetrate the inner sleeve 32 in the radial direction are formed over the entire length in the front-rear direction, excluding the front end portion, at intervals in the circumferential direction.

A rear end portion of the second body tube 33 is inserted between an inner peripheral surface of a front portion of the outer sleeve 31 and an outer peripheral surface of a front portion of the inner sleeve 32. The second body tube 33 protrudes forward from the outer sleeve 31 and the inner sleeve 32. A male screw portion (not shown) is formed on an outer peripheral surface of the rear end portion of the second body tube 33, and a female screw portion (not shown) that is screwed to the male screw portion of the second body tube 33 is formed on an inner peripheral surface of a front portion of the outer sleeve 31.

The front portion of the circumferential wall portion 115b of the rear fixation body 115 is fitted and fixed into the inner sleeve 32 through a rear end opening of the outer sleeve 31. By tightening the outer sleeve 31 onto the second body tube 33, a portion of the inner sleeve 32, excluding the front end portion, is reduced in diameter, and the inner peripheral surface of the inner sleeve 32 clamps the outer peripheral surface of the front portion of the circumferential wall portion 115b of the rear fixation body 115.

A front portion of the second body tube 33 is inserted into the first body tube 23 to be movable in the front-rear direction. A rear portion of the second body tube 33 protrudes rearward from the first body tube 23. An outer diameter of the rear portion of the second body tube 33 is larger than an outer diameter of the front portion of the second body tube 33 and is the same as an outer diameter of the first body tube 23. A size of the outer diameter of the rear portion of the second body tube 33 is not limited to the present embodiment and may be appropriately changed.

The outer tube 13 extends in the front-rear direction and is rotatably fitted around the first tubular member 11 and the second tubular member 12 about the central axis O. In the example shown in the drawing, the outer tube 13 is fitted around the first body tube 23 and the second body tube 33.

The outer tube 13 is provided on any one of the first tubular member 11 and the second tubular member 12 in a state where movement in the front-rear direction is restricted. A first guide portion 25 is formed on an inner peripheral surface of the outer tube 13 and an outer peripheral surface of the other tubular member of the first tubular member 11 and the second tubular member 12. The first guide portion 25 moves the other tubular member in the front-rear direction with respect to the one tubular member in such a manner that a distance in the front-rear direction between the front fixation body 113 and a front end portion of the support tube body 114 is reduced, in accordance with relative rotation of the outer tube 13 and the other tubular member about the central axis O.

In the example shown in the drawing, the outer tube 13 is provided on the first tubular member 11 in a state where movement in the front-rear direction is restricted, and the first guide portion 25 is formed on the inner peripheral surface of the outer tube 13 and the outer peripheral surface of the second tubular member 12.

The first guide portion 25 includes a first engagement protrusion 26 and a first guide groove 27 that extends around the central axis O and in which the first engagement protrusion 26 is movably accommodated around the central axis O.

In the example shown in the drawing, two first engagement protrusions 26 are formed on the inner peripheral surface of the outer tube 13 at intervals in the circumferential direction. The two first engagement protrusions 26 face each other in the radial direction. The first engagement protrusion 26 is provided at a rear end portion of the outer tube 13.

The two first guide grooves 27 are formed on the outer peripheral surface of the second body tube 33 of the second tubular member 12. The first guide groove 27 spirally extends around the central axis O. The first guide groove 27 is formed on the outer peripheral surface of a rear portion of the second body tube 33 that protrudes rearward from the first body tube 23. The first engagement protrusions 26 are individually accommodated in the two first guide grooves 27. The first engagement protrusion 26 is located in a rear end portion of the first guide groove 27 in a standby state before the guide wire 100 is operated. A front end portion of the first guide groove 27 may be open toward the front.

Here, a second guide portion 35 that allows relative movement in the front-rear direction is formed in the first tubular member 11 and the second tubular member 12 in a state where the first tubular member 11 and the second tubular member 12 are restricted from rotating relative to each other around the central axis O.

The second guide portion 35 includes a second engagement protrusion 36 and a second guide groove 37 that extends in the front-rear direction and in which the second engagement protrusion 36 is movably accommodated in the front-rear direction. The first guide portion 25 and the second guide portion 35 are separated from each other in the front-rear direction.

In the example shown in the drawing, two second engagement protrusions 36 are formed on the outer peripheral surface of a front portion of the second body tube 33 that is located in the first body tube 23 at intervals in the circumferential direction. The second engagement protrusion 36 may be one. The second engagement protrusion 36 is provided at a front end portion of the second body tube 33. The two second engagement protrusions 36 are provided at positions facing each other in the radial direction. An outer end portion of the second engagement protrusion 36 in the radial direction is located at the same position in the radial direction or on the inner side in the radial direction with respect to the outer peripheral surface of the first body tube 23. In the front end portion of the second body tube 33, a notch portion 33a that penetrates in the radial direction and is open to a front end opening edge of the second body tube 33 is formed at each portion located between the second engagement protrusions 36 adjacent to each other in the circumferential direction. As a result, the front end portion of the second body tube 33 is formed to be elastically deformable in the radial direction in a direction in which the two second engagement protrusions 36 approach each other.

The second guide groove 37 is formed on an inner peripheral surface of the first body tube 23 of the first tubular member 11 and extends in the front-rear direction. The second guide groove 37 penetrates the first body tube 23 in the radial direction. The second guide groove 37 need not penetrate the first body tube 23 in the radial direction. The two second guide grooves 37 are formed on the first body tube 23 at intervals in the circumferential direction. The two second guide grooves 37 face each other in the radial direction. The second engagement protrusions 36 are individually accommodated in the two second guide grooves 37. The second engagement protrusion 36 is located in a front end portion of the second guide groove 37 in a standby state before the guide wire 100 is operated.

The rear end portion of the first body tube 23 of the first tubular member 11 is inserted into the front end portion of the outer tube 13.

Two third engagement protrusions 38 that protrude toward the inner side in the radial direction are formed on an inner peripheral surface of the front end portion of the outer tube 13 at intervals in the circumferential direction. The two third engagement protrusions 38 face each other in the radial direction. The third engagement protrusions 38 are formed in a protruding shape extending in the circumferential direction. In the front portion of the outer tube 13, a notch portion 13a that penetrates in the radial direction and is open to a front end opening edge of the outer tube 13 is formed at each portion located between the third engagement protrusions 38 adjacent to each other in the circumferential direction. As a result, the front portion of the outer tube 13 is formed to be elastically deformable in the radial direction in a direction in which the two third engagement protrusions 38 are separated from each other.

A circumferential groove 39 extending continuously over the entire length in the circumferential direction is formed on the outer peripheral surface of the rear end portion of the first body tube 23. The third engagement protrusions 38 are accommodated in the circumferential groove 39 in a state where the movement in the front-rear direction is restricted, so as to be rotatable about the central axis O. The third engagement protrusions 38 are engaged with the circumferential groove 39, so that the outer tube 13 is provided to be rotatable about the central axis O in a state where the movement in the front-rear direction is restricted with respect to the first tubular member 11.

The third engagement protrusions 38 and the circumferential groove 39 are provided between the first guide portion 25 and the second guide portion 35 along the front-rear direction.

As described above, with the operation member 1 for a guide wire according to the present embodiment, in a case where the outer tube 13 provided on the first tubular member 11 in a state where movement in the front-rear direction is restricted is rotated around the central axis O, the second tubular member 12 moves rearward with respect to the first tubular member 11 as shown in FIG. 2 by the first guide portion 25 formed on the inner peripheral surface of the outer tube 13 and on the outer peripheral surface of the second tubular member 12. In this case, the operation wire 112 is pulled rearward, so that the distance between the front fixation body 113 and the front end portion of the support tube body 114 in the front-rear direction is shortened, and the flexible tube body 111 is compressed and deformed.

As a result, for example, when an eccentric load is generated in the flexible tube body 111 in a case where the flexible tube body 111 is a coil spring, or in a case where the central axes of each of the flexible tube body 111 and the operation wire 112 do not coincide with one another, for example, as shown by a two-dot chain line in FIG. 2, the flexible tube body 111 is compressed and deformed while being bent in a specific direction, and the outer tube 13 is operated outside the body to select a blood vessel at a blood vessel bifurcation while bending the front end portion of the guide wire 100 in the body, so that the guide wire 100 can enter a target blood vessel. As a result, it is possible to reduce reliance on a physician's tactile sense during manipulation when advancing the guide wire 100 along the blood vessel to reach a chronic total occlusion lesion or the like. In addition, it is possible to eliminate a need to finely adjust a curvature of the front end portion of the guide wire outside the body by repeatedly inserting and removing the guide wire into and from the blood vessel.

Further, when the outer tube 13 is rotated around the central axis O, the distance between the front fixation body 113 and the front end portion of the support tube body 114 in the front-rear direction is shortened, and the flexible tube body 111 is compressed and deformed in the front-rear direction, the bending stiffness of the flexible tube body 111 is increased. Therefore, the bending stiffness of the flexible tube body 111 can be changed by operating the outer tube 13 outside the body in a state where the guide wire 100 is inserted into the blood vessel.

As a result, when penetrating the chronic total occlusion lesion or the like with the guide wire 100, the bending stiffness of the flexible tube body 111 can be kept low until the guide wire 100 enters the blood vessel and reaches the chronic total occlusion lesion or the like in the blood vessel, and the outer tube 13 can be rotated around the central axis O when the chronic total occlusion lesion or the like is punctured with the guide wire 100 so as to penetrate the chronic total occlusion lesion or the like, whereby the bending stiffness of the flexible tube body 111 can be increased, and the outer tube 13 can be rotated in reverse after the chronic total occlusion lesion or the like is penetrated to thereby return the bending stiffness of the flexible tube body 111 to the original low state. Therefore, it is possible to eliminate the need to repeatedly insert, remove, and exchange guide wires having different bending stiffnesses into and from a blood vessel or the like in order to penetrate the chronic total occlusion lesion or the like.

As described above, it is possible to shorten an operation time.

In the above process, the movement of the outer tube 13 in the front-rear direction with respect to the first tubular member 11 is restricted, and the first guide portion 25 is formed on the inner peripheral surface of the outer tube 13 and the outer peripheral surface of the second tubular member 12. Therefore, when the outer tube 13 is rotated around the central axis O, the movement of the outer tube 13 in the front-rear direction with respect to the first tubular member 11 is restricted, and the second tubular member 12 can be reliably moved in the front-rear direction with respect to the first tubular member 11, so that the front end portion of the guide wire 100 can be accurately deformed into a desired shape.

The design of bending the flexible tube body 111 in a specific direction when the outer tube 13 is rotated around the central axis O can be performed, for example, by adjusting an eccentric amount, an eccentric direction, a tilt angle, and the like of the central axis of each of the flexible tube body 111 and the operation wire 112, or by adjusting an eccentric amount, an eccentric direction, and the like of a load generated in the flexible tube body 111 when a compression load is applied in a case where the flexible tube body 111 is a coil spring. The latter adjustment can be performed, for example, by adjusting a spring shape such as the number of turns of the coil spring (relative circumferential positions of both end portions of the wire material), a pitch angle of the coil spring, a pitch of the coil spring, a wire diameter (diameter of the wire material), a gap between the wires, a coil outer diameter, and a support method of both end portions of the coil spring (for example, inclinations of both end portions of the wire material).

The first guide portion 25 and the second guide portion 35 are separated from each other in the front-rear direction, the first engagement protrusion 26 and the second engagement protrusion 36 are individually accommodated in the first guide groove 27 and the second guide groove 37, and are not integrally accommodated in the first guide groove 27 and the second guide groove 37. Therefore, it is possible to suppress the amount of protrusion of each of the first engagement protrusion 26 and the second engagement protrusion 36. In addition, compared to a configuration in which the first engagement protrusion 26 or the second engagement protrusion 36 is integrally accommodated in the first guide groove 27 and the second guide groove 37, the first engagement protrusion 26 and the second engagement protrusion 36 can be easily and individually accommodated in the first guide groove 27 and the second guide groove 37, without excessively radially elastically deforming the first body tube 23 or the second body tube 33 during assembly, and the stiffness of each of the first body tube 23 and the second body tube 33 can be easily ensured.

The technical scope of the present invention is not limited to the above embodiment, and various modifications can be made without departing from the spirit of the present invention.

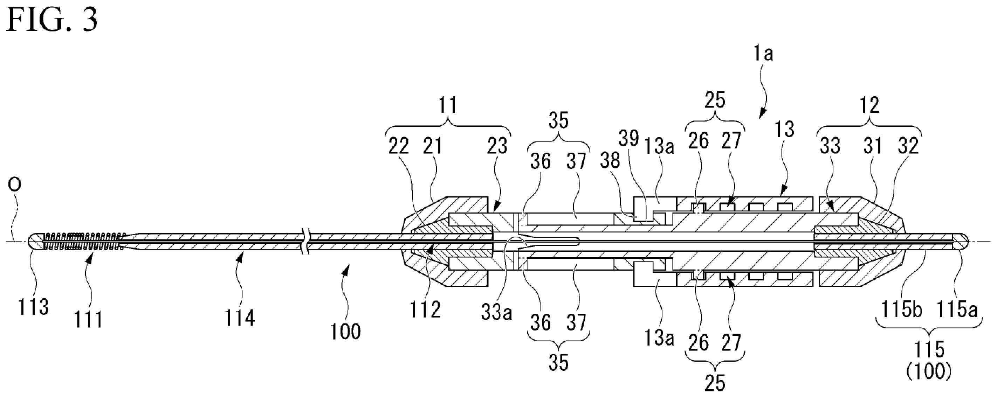

For example, as in an operation member 1a for a guide wire shown in FIG. 3, a configuration in which the first engagement protrusion 26 is formed on the outer peripheral surface of the rear portion of the second body tube 33 of the second tubular member 12 that protrudes rearward from the first body tube 23, instead of the outer tube 13, and the first guide groove 27 is formed on the inner peripheral surface of the outer tube 13, instead of the rear portion of the second body tube 33, as the first guide portion 25 may be adopted.

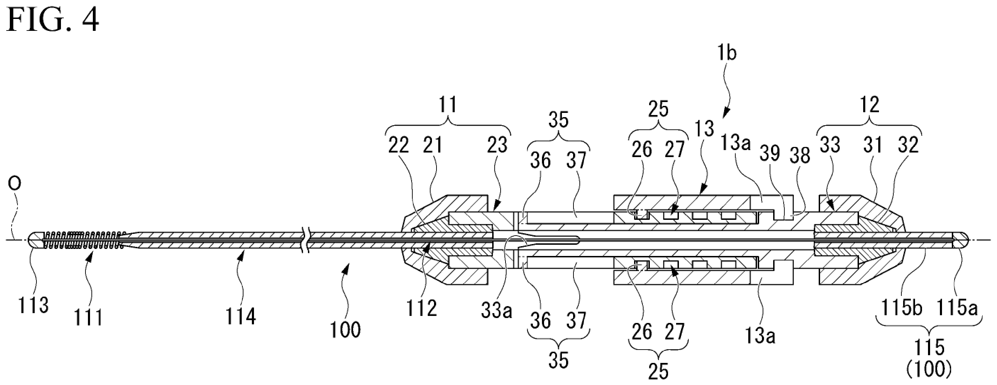

As in an operation member 1b for a guide wire shown in FIG. 4, the outer tube 13 may be provided on the second tubular member 12 in a state where the movement in the front-rear direction is restricted, and the first guide portion 25 may be formed on the inner peripheral surface of the outer tube 13 and the outer peripheral surface of the first tubular member 11.

Hereinafter, the operation member 1b for a guide wire will be specifically described with reference to FIG. 4.

The first engagement protrusion 26 of the first guide portion 25 is provided at the front end portion of the outer tube 13, and the first guide groove 27 of the first guide portion 25 is formed on the outer peripheral surface of the first body tube 23 of the first tubular member 11. The first guide groove 27 is formed on the outer peripheral surface of the rear portion of the first body tube 23 that is inserted into the outer tube 13. In the example shown in the drawing, the rear portion of the first body tube 23 is inserted into a portion of the outer tube 13 that is located in front of the rear end portion. In a standby state before the guide wire 100 is operated, the first engagement protrusion 26 is located in a front end portion of the first guide groove 27.

The third engagement protrusion 38 is formed on the inner peripheral surface of the rear end portion of the outer tube 13, and the notch portion 13a is provided at the rear portion of the outer tube 13. The circumferential groove 39 is formed on the outer peripheral surface of a rear portion of the second body tube 33 that protrudes rearward from the first body tube 23. The third engagement protrusion 38 and the circumferential groove 39 are located behind the first guide portion 25 and the second guide portion 35.

In the above configuration, in a case where the outer tube 13 provided on the second tubular member 12 in a state where movement in the front-rear direction is restricted is rotated around the central axis O, the first tubular member 11 moves forward with respect to the second tubular member 12 by the first guide portion 25 formed on the inner peripheral surface of the outer tube 13 and on the outer peripheral surface of the first tubular member 11. In this case, the support tube body 114 is pushed forward by the first tubular member 11, so that the distance between the front fixation body 113 and the front end portion of the support tube body 114 in the front-rear direction is shortened, and the flexible tube body 111 is compressed and deformed.

As described above, the same action and effect as those of the embodiment described above are exhibited.

Further, in the present modification example, since the first tubular member 11 moves forward with respect to the second tubular member 12 in accordance with the rotation of the outer tube 13 around the central axis O with respect to the first tubular member 11, the flexible tube body 111 can be deformed without changing the position of the front fixation body 113 in the front-rear direction.

In the operation member 1 for a guide wire shown in FIGS. 1 and 2, a configuration in which the front and rear are reversed, the second tubular member 12 (first tubular member) is fixed to the rear end portion of the support tube body 114, and the first tubular member 11 (second tubular member) is connected to the portion of the operation wire 112 that protrudes rearward from the support tube body 114 may be adopted. In this case, the same action and effect as those of the operation member 1b for a guide wire shown in FIG. 4 are exhibited.

In addition, it is possible to appropriately replace the constituent elements in the above embodiment with well-known constituent elements without departing from the spirit of the present invention. Additionally, the above-described embodiment and the modification examples may be combined with each other as appropriate.

### INDUSTRIAL APPLICABILITY

According to the present invention, it is possible to provide an operation member for a guide wire that can accurately deform a front end portion of the guide wire into a desired shape.

### REFERENCE SIGNS LIST

1, 1a, 1b Operation member for guide wire
11 First tubular member
12 Second tubular member
13 Outer tube
25 First guide portion
26 First engagement protrusion
27 First guide groove
35 Second guide portion
36 Second engagement protrusion
37 Second guide groove
111 Flexible tube body
112 Operation wire
113 Front fixation body (fixation body)
114 Support tube body
100 Guide wire
O Central axis (axis)

## Claims

1. An operation member for a guide wire, the operation member being mounted on a guide wire including
a flexible tube body extending in a front-rear direction,
an operation wire extending in the front-rear direction and provided inside the flexible tube body,
a fixation body to which a front end edge of the operation wire is fixed, and
a support tube body that is provided on a rear side of the fixation body, that sandwiches the flexible tube body in the front-rear direction between the fixation body and the support tube body, and that has the operation wire inserted therethrough, the operation member comprising:
a first tubular member that is fixed to a rear end portion of the support tube body and that extends in the front-rear direction;
a second tubular member that is connected to a portion of the operation wire that protrudes rearward from the support tube body, the second tubular member extending in the front-rear direction; and
an outer tube that is rotatably fitted around the first tubular member and the second tubular member about an axis and that extends in the front-rear direction, the outer tube extending in the front-rear direction,
wherein the first tubular member and the second tubular member are provided to be movable relative to each other in the front-rear direction and to be restricted from rotating relative to each other around the axis,
the outer tube is provided on any one of the first tubular member and the second tubular member in a state where movement in the front-rear direction is restricted, and
a first guide portion is formed on an inner peripheral surface of the outer tube and on an outer peripheral surface of the other one of the first tubular member and the second tubular member, the first guide portion being configured, in accordance with relative rotation of the outer tube and the other tubular member about the axis, to move the other tubular member in the front-rear direction relative to the one tubular member such that a distance in the front-rear direction between the fixation body and a front end portion of the support tube body changes.

2. The operation member for a guide wire according to claim 1,
wherein the first guide portion includes a first engagement protrusion and a first guide groove that extends around the axis and in which the first engagement protrusion is movably accommodated around the axis, and
a second guide portion that allows relative movement in the front-rear direction is formed in the first tubular member and the second tubular member in a state where the first tubular member and the second tubular member are restricted from rotating relative to each other around the axis,
the second guide portion includes a second engagement protrusion and a second guide groove that extends in the front-rear direction and in which the second engagement protrusion is movably accommodated in the front-rear direction, and
the first guide portion and the second guide portion are separated from each other in the front-rear direction.
